# EUROPEAN PATENT SPECIFICATION

(11) **EP 4 512 462 B1**
(45) Date of publication and mention of the grant of the patent: **27.05.2026**
(21) Application number: 24196099.6
(22) Date of filing: 23.08.2024
(51) Int. Cl.: A61N 1/05, A61N 1/375, A61N 1/372

(54) **BIOSTIMULATOR HAVING PACING EXTENSION**
BIOSTIMULATOR MIT STIMULATIONSVERLÄNGERUNG
BIOSTIMULATEUR COMPORTANT UNE EXTENSION DE STIMULATION

(30) Priority: 25.08.2023 US 202363534786 P; 22.08.2024 US 202418812881
(43) Date of publication of application: 26.02.2025
(73) Proprietor: Pacesetter, Inc., Sylmar, CA 91342 (US)
(72) Inventor: Arnar, Bernhard, Sylmar, CA 91342 (US); Jackson, Aaron, Sylmar, CA 91342 (US)
(74) Representative: Barker Brettell Sweden AB

(56) References cited:
- EP-A1- 4 252 832
- EP-A2- 4 197 589
- WO-A1-2018/165377
- US-A1- 2016 310 723

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricle apex. More particularly, a pacing site for LBB pacing is typically below the His-bundle, on the interventricular septal wall near the tricuspid valve and pulmonary artery outflow track.

EP 4 252 832 A1 discloses a biostimulator and a biostimulator system for septal pacing. The biostimulator includes an articulation to allow an electrode axis of a pacing electrode to be directed differently than a housing axis of a housing. The housing contains electrical circuitry that is electrically connected to the pacing electrode. The differently directed axes allow the pacing electrode to affix to target tissue of an interventricular septal wall of a heart when the housing of the biostimulator is located near an apex of the heart. The articulation can include a flexible portion of an extension, a hinge, or a tether.

US 2016/310723 A1 discloses an implantable pacemaker system including a housing having a proximal end and a distal end. A control electronics subassembly defines the housing proximal end, and a battery subassembly defines the housing distal end. A distal fixation member extends from the housing distal end for fixing the housing distal end at an implant site. A pacing extension extends from the housing proximal end and carries a pacing cathode electrode. The pacing extension extends the pacing cathode electrode to a pacing site that is spaced apart from the implant site when the pacemaker is deployed in a patient's body.

### SUMMARY

Existing leadless pacemakers may not fit, or may interfere with heart structures, when placed at the pacing site for left bundle branch (LBB) pacing. More particularly, existing leadless pacemakers having bodies that are long and rigid and, when implanted at the interventricular septal wall, could extend into contact with the cardiac tissue of a ventricular free wall or the tricuspid valve. The long and rigid body of existing leadless pacemakers could also become tangled within chordae tendinae. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator that can be engaged to the interventricular septal wall to pace the LBB without interfering with adjacent structures of the heart and with minimal trauma during attachment and retrieval.

A biostimulator is described. In an embodiment, the biostimulator includes a housing, an electrical extension, and a pacing extension body. The housing may include an electronics compartment containing pacing circuitry. The electrical extension may include an elongated electrical conductor. The elongated electrical extension may extend from a proximal extension end at the housing to a distal extension end. The electrical extension is flexible to bend and move the distal extension end relative to the proximal extension end. The pacing extension body may be mounted on the distal end of the electrical extension. The pacing extension body may include a pacing electrode electrically coupled to the pacing circuitry through the elongated electrical conductor, a free-rotating fixation helix, and a drive mechanism to transmit torque to rotate the fixation helix relative to the electrical extension and the housing. For example, the electrical extension may be flexible such that the pacing electrode may be affixed on a different axis from the housing. Accordingly, the pacing electrode can engage target tissue on an upper portion of the interventricular septal wall while the housing can be directed toward the ventricular apex without interfering with adjacent structures of the heart. Additionally, the fixation helix and drive mechanism of the pacing extension body may allow for greater depth control of the electrode in the septal wall and prevent any spinning of the lead or device, further reducing the likelihood of interference with adjacent structures.

In some examples, the pacing extension body includes a port providing access for insertion of a drive tool to apply a torque to the drive mechanism of the pacing body. Accordingly, a separate drive tool may be inserted at the port to drive the fixation helix and to affix the pacing electrode within the septal wall tissue. In some examples, the drive tool may be integrated in the pacing extension body. In some examples, the fixation mechanism includes a free turning fixation helix within or incorporated with the pacing electrode, providing additional control of fixation depth and independent attachment of the pacing electrode. In some embodiments, the pacing extension body includes a release and retrieve mechanism.

A biostimulator system is described. In an embodiment, the biostimulator system includes a biostimulator transport system. The biostimulator can be mounted on the biostimulator transport system to carry the biostimulator to or from the target anatomy.

### BRIEF DESCRIPTION OF THE DRAWINGS

The novel features of the invention are set forth with particularity in the claims that follow. The present invention is defined by the appended claims. Aspects, embodiments and examples described hereinafter are only of exemplary nature and are presented for better understanding the present invention. A better understanding of the features and advantages of the present disclosure will be obtained by reference to the following detailed description that sets forth illustrative embodiments, in which the principles of the disclosure are utilized, and the accompanying drawings.
FIG. 1 is a diagrammatic cross section of a patient heart illustrating an example implantation of a biostimulator in a target anatomy, in accordance with an embodiment.
FIG. 2 is a side view of a pacing extension body of a biostimulator having an electrical extension and a free-rotation fixation helix, in accordance with an embodiment.
FIG. 3 is a side view of a pacing extension body of a biostimulator having an electrical extension and a free-rotation fixation helix with a drive tool, in accordance with an embodiment.
FIG. 4A is a side view of a biostimulator having an electrode extension and free-rotating fixation helix in a transport system, in accordance with an embodiment.
FIG. 4B is another side view of a biostimulator having an electrode extension and free-rotating fixation helix in a transport system, in accordance with an embodiment.
FIG. 5 is a diagrammatic cross section of a patient heart illustrating an example implantation of a biostimulator in a target anatomy, in accordance with an embodiment.
FIG. 6 is a side view of a pacing extension body of a biostimulator having an integrated drive mechanism and a free-rotating fixation helix, in accordance with an embodiment.
FIG. 7A is a side view of a biostimulator having a pacing extension body with an integrated drive mechanism and free-rotating fixation helix in a transport system, in accordance with an embodiment.
FIG. 7B is another side view of a biostimulator having a pacing extension body with an integrated drive mechanism and free-rotating fixation helix in a transport system, in accordance with an embodiment.
FIG. 8 is a side view of a biostimulator including an angled electrode extension with a free-rotating fixation helix, in accordance with an embodiment.
FIG. 9 is a side view of a biostimulator including an angled electrode extension with a free-rotating fixation helix in a transport system, in accordance with an embodiment.
FIG. 10 is a cross-sectional view of a pacing extension body with a free-rotating fixation helix, in accordance with an embodiment.
FIG. 11 is a perspective view of a pacing extension body with a free-rotating fixation helix with attachment and detachment button, in accordance with an embodiment.
FIG. 12 is a side view of a pacing extension body including a retrieval component and a retrieval system, in accordance with an embodiment.
FIG. 13 is a side view of a pacing extension body including a retrieval component engaged by a retrieval system, in accordance with an embodiment.
FIG. 14 is a flowchart of a method of implanting a biostimulator for septal pacing.

### DETAILED DESCRIPTION

Embodiments describe a biostimulator and a biostimulator system for septal pacing. The biostimulator may, however, be used in other applications, such as deep brain stimulation. Thus, reference to the biostimulator as being a cardiac pacemaker for septal pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a housing, an electrical extension, and a pacing extension body. The housing may include an electronics compartment containing pacing circuitry. The electrical extension may include an elongated electrical conductor. The elongated electrical extension may extend from a proximal extension end at the housing to a distal extension end. The pacing extension body may be mounted on the distal end of the electrical extension. The pacing extension body may include a pacing electrode electrically coupled to the pacing circuitry through the elongated electrical conductor, a fixation mechanism, and a drive mechanism, e.g., a torque drive, to transmit torque to the fixation mechanism. For example, the electrical extension may be flexible such that the pacing electrode may be affixed on a different axis from the housing.

In some embodiments, the pacing extension body or the electrical extension may include a port to allow the torque drive (e.g., an external mechanism such as a wrench or other tool to transmit torque) to be inserted into the pacing extension body to engage the fixation mechanism (e.g., via a socket or other coupling in which the torque drive may be inserted). Such an embodiment(s) may have a small form factor, simplifying the delivery device (e.g., catheter) and reducing a likelihood of contact with adjacent structures. In some embodiments, a torque drive mechanism (e.g., a socket connected to the fixation helix) may be incorporated into the pacing extension body and extend outward from the pacing extension body.

Accordingly, the free-rotating fixation helix provides for separate and independent attachment of the pacing electrode and the housing which prevents spinning of the lead or the device during attachment. Additionally, the free-rotating fixation helix allows greater depth control of the pacing electrode as well as the potential for multiple attachments to obtain a sufficient position of the pacing electrode to engage nerve structures (e.g., left bundle branch area) of the ventricular septal wall with minimal trauma.

Additionally, because the electrical extension provides for separate movement of the pacing extension body and the housing of the biostimulator, when the fixation element is anchored in a septal wall of a heart, the housing can be located in the ventricular apex without interfering with a heart valve or an outer heart wall opposite to the septal wall. The biostimulator therefore fits well within the limited space of the target heart chamber. A biostimulator system is described that can transport the biostimulator to or from a pacing site at the septal wall.

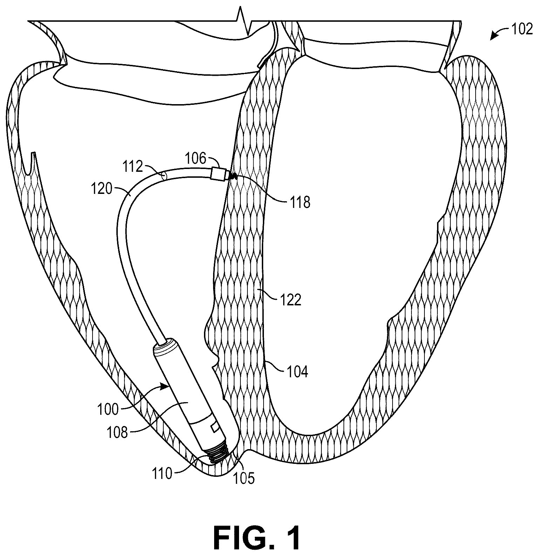

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to the septum, and one or more elements, such as a pacing electrode 106, can pierce the interventricular septal wall 104 of the septum to engage and anchor the biostimulator 100 to the tissue. For example, a fixation helix 118 may rotate (e.g., when torque is applied) to affix the pacing electrode 106 in the septal wall 104. The fixation helix 118 may be attached to the pacing electrode 106 in a manner providing for free-rotation of the fixation helix 118 with respect to the pacing electrode 106. The pacing electrode 106 may be a separate component from the fixation helix 118 or the pacing electrode 106 may include the fixation helix 118 (e.g., the fixation helix 118 may be electrically coupled to the pacing electrode 106). Similarly, a housing 108 and/or an anchor 110 can be delivered into the ventricular apex 105.

The pacing electrode 106 can have an electrode axis, which is directed toward, e.g., normal to, the septal wall when the pacing electrode 106 is affixed to the septal wall. Similarly, the housing 108 can have a housing axis, which is directed toward, e.g., oblique to, an apex wall of the ventricular apex 105 when the housing 108 is located therein. When the pacing electrode 106 is affixed to the interventricular septal wall 104, and the housing 108 is located at the ventricular apex 105, the electrode axis can extend in a different direction than the housing axis. For example, the electrode axis can extend in a direction that is transverse or oblique to a direction of the housing axis. Accordingly, the pacing electrode 106 can be located to effectively probe and pace the left bundle branch 122, while the housing 108 can be placed in a safe and non-obstructive location within the heart chamber.

The non-coaxial relationship of the electrode axis and the housing axis, which allows for safe and non-obstructive placement of the pacing electrode 106 and the housing 108, may be provided by an electrical extension 120 of the biostimulator 100. The electrical extension 120 can be located between the pacing electrode 106 and the housing 108. For example, the electrical extension 120 may include an elongated electrical conductor to couple the pacing electrode 106 with the electronics of the housing 108. The electrical extension 120 may extend from a proximal extension end at the housing to a distal extension end where the pacing electrode 106 mounted. More particularly, electrical extension 120 may include a flexible tubing, conduit, casing, molding, etc., to provide electrical isolation or encasing of the elongated electrical conductor. In some embodiments, the electrical extension 120 may include a pacing extension body at a distal end. The pacing extension body may be a segment of an insulative sheath that covers the electrical extension 120. In some examples, the pacing extension body may be comprised of the same or a different material than the other portions of the electrical extension 120. For example, the electrical extension 120 may be comprised of a flexible material while the pacing extension body may be comprised of a hard inflexible material (e.g., as a single component). The pacing extension body of the electrical extension 120 may include a drive port 112 in which a drive tool (e.g., a torque drive) may be inserted into the electrical extension 120 to engage and apply a torque to the fixation helix 118 to rotate the fixation helix 118. Because the fixation helix 118 is freely rotating, the torque is only applied to the fixation helix 118 and not to the electrical extension 120 or the housing 108. Accordingly, there is no unintended rotation or movement of the biostimulator during attachment of the pacing electrode 106 via the fixation helix 118.

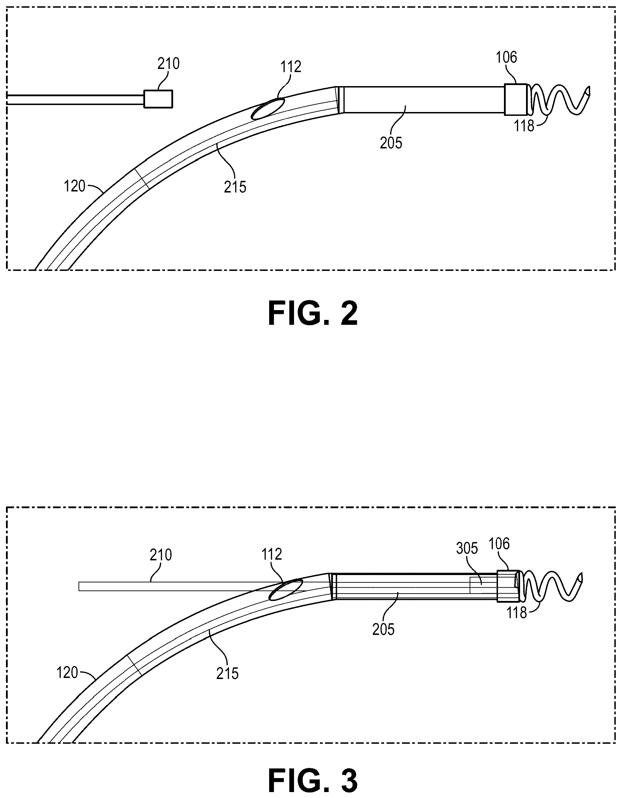

In some embodiments, as described in more detail below with respect to FIG. 2, a pacing extension body may be mounted on the distal extension end of the electrical extension 120. The pacing extension body may include the pacing electrode 106, the fixation helix 118, and the drive port 112. The pacing extension body may provide a structure to support the free-rotation of the fixation helix 118 as well as the drive port and drive mechanism for engaging the fixation helix 118 using an drive tool.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems, or biostimulator transport systems. Examples of transport systems are described below. In some implementations of biostimulator systems, a leadless pacemaker is attached, connected to, or otherwise mounted on a distal end of a catheter of the biostimulator transport system. The leadless pacemaker is thereby advanced intravenously into or out of the heart 102. The transport system can include features to engage the leadless pacemaker to allow fixation of the leadless pacemaker to tissue. For example, in implementations where the leadless pacemaker includes an active engaging mechanism, such as a helical fixation element, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum of the heart 102, the pacing electrode 106 may be positioned for deep septal pacing at a target bundle branch 122 in the septum. For example, an active electrode of the pacing element can be positioned at the left bundle branch 122 in the septum. The biostimulator 100 may deliver pacing impulses through the pacing electrode 106 to the bundle branch(es).

Referring to FIG. 2, a side view of a pacing extension body of a biostimulator having an electrical extension and a free-rotation fixation helix is shown in accordance with an embodiment. A pacing extension body 205 may be incorporated at a distal end of the electrical extension 120 of the biostimulator 100. The pacing extension body 205 may include a pacing electrode 106 electrically coupled to pacing circuitry of the biostimulator housing 108 via an elongated electrical conductor 215 of the electrical extension 120. The pacing extension body 205 or the electrical extension 120 may additionally include a drive port 112 through which a torque drive 210 may be inserted to engage the fixation helix 118. As provided above, the fixation helix 118 may be freely rotating within the pacing extension body 205 and within the pacing electrode 106. For example, the fixation helix 118 may be rotatable by the torque drive 210 without applying a torque on the pacing electrode 106 or the electrical extension 120.

Referring to FIG. 3, a side view of a pacing extension body of a biostimulator having an electrical extension and a free-rotation fixation helix with a drive tool is shown in accordance with an embodiment. As depicted, the torque drive 210 may be inserted through the drive port 112 of the electrical extension 120 or pacing extension body 205 to engage the fixation helix 118. For example, the torque drive 210 may engage the fixation helix 118 at engagement point 305 within the pacing extension body 205. The torque drive 210 may apply a torque to the fixation helix 118 but not the pacing electrode 106, the electrical extension 120, or the elongated electrical conductor 215.

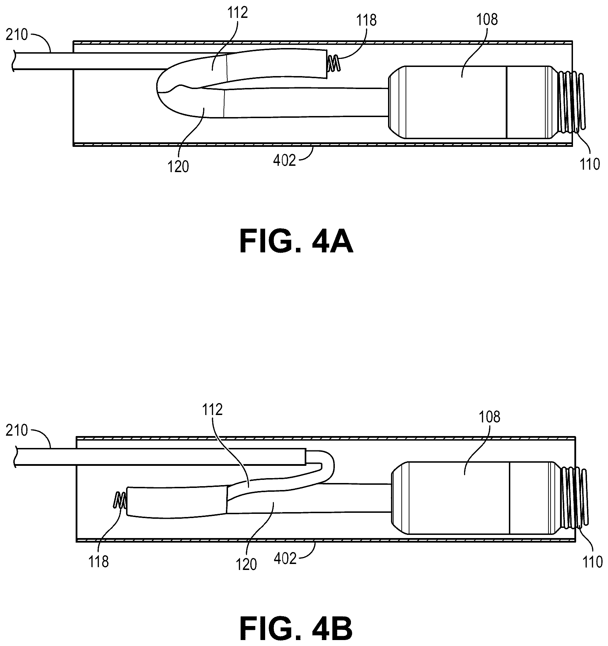

Referring to FIG. 4A, a side view of a biostimulator having an electrode extension and free-rotating fixation helix in a transport system is shown in accordance with an embodiment. To deploy the biostimulator 100, the biostimulator 100 may be inserted within a transport system 402, such as within a catheter that may be used to deploy the biostimulator intravenously to the heart. To incorporate the biostimulator with the electrical extension 120 and housing 108 within the transport system 402, the electrical extension 120 may be folded, articulated, bent, or otherwise angled within the transport system 402. In some examples, the torque drive 210 may be inserted within the drive port 112 prior to deployment to reduce space required within the transport system 402 to fit the biostimulator 100. In some embodiments, the transport system 402 may be used to first attach the housing 108 (e.g., at a ventricular apex 105) after which the biostimulator 100 may be removed from the transport system 402. The fixation helix 118 and pacing electrode 106 may then be aligned and attached (e.g., at a septal wall 104). The torque drive 210 may be inserted in the port 112 of the electrical extension 120 to apply a torque and rotate the fixation helix 118 until an appropriate depth of the pacing electrode 106 is reached within the septal wall.

Referring to FIG. 4B, a side view of a biostimulator having an electrode extension and free-rotating fixation helix in a transport system is shown in accordance with an embodiment. Similar to FIG. 4B, the biostimulator 100 may be inserted within a transport system 402, such as within a catheter that may be used to deploy the biostimulator intravenously to the heart. However, as depicted in FIG. 4B the torque drive 210 may be folded, articulated, bent, or otherwise angled (e.g., at or near 180 degrees) within the transport system 402 while the electrical extension 120 remains unbent within the transport system 402.

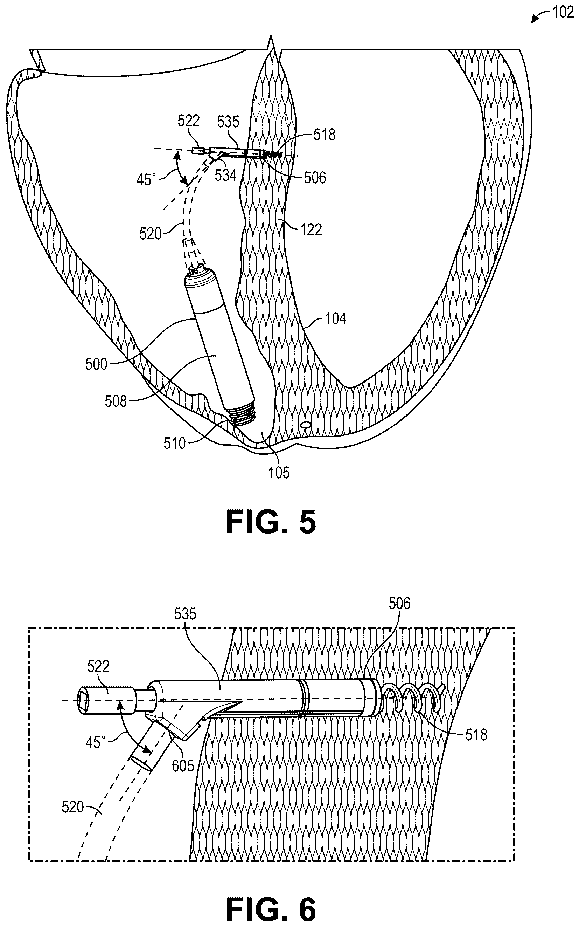

Referring to FIG. 5, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 500. The biostimulators 500 can be implanted in a patient heart 102, and can be leadless (and thus, may be leadless cardiac pacemakers). Each biostimulator 500 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 500 can be delivered to the septum, and one or more elements, such as a pacing electrode 506, can pierce the interventricular septal wall 104 of the septum to engage and anchor the biostimulator 500 to the tissue. For example, a fixation helix 518 may rotate (e.g., when torque is applied) to affix the pacing electrode 506 in the septal wall 104. The fixation helix 518 may be coupled to the pacing electrode 506 in a manner providing for free rotation of the fixation helix 518 with respect to the pacing electrode 506. The pacing electrode 506 may be a separate component from the fixation helix 118 or the pacing electrode 506 may include the fixation helix 118 (e.g., the fixation helix 118 may be electrically coupled to the pacing electrode 506). Similarly, a housing 508 and/or an anchor 510 can be delivered into the ventricular apex 105. In some embodiments, the torque drive 522 may be integrated in the pacing extension body 535 and may be coupled directly with the fixation helix 518. For example, the torque drive 522 may also be freely rotatable within the pacing extension body 535 and may engage the fixation helix 518 or may be directly attached to the fixation helix 518. Because the fixation helix 518 is freely rotating, the torque is only applied to the fixation helix 518 and not to the electrical extension 520 or the housing 508. Accordingly, there is no unintended rotation or movement of the biostimulator during attachment of the pacing electrode 506 via the fixation helix 518.

The pacing electrode 506 can have an electrode axis, which is directed toward, e.g., normal to, the septal wall when the pacing electrode 506 is affixed to the septal wall. Similarly, the housing 508 can have a housing axis, which is directed toward, e.g., oblique to, an apex wall of the ventricular apex 105 when the housing 508 is located therein. When the pacing electrode 506 is affixed to the interventricular septal wall 104, and the housing 508 is located at the ventricular apex 105, the electrode axis can extend in a different direction than the housing axis. For example, the electrode axis can extend in a direction that is transverse or oblique to a direction of the housing axis. Accordingly, the pacing electrode 506 can be located to effectively probe and pace the left bundle branch 122, while the housing 508 can be placed in a safe and non-obstructive location within the heart chamber.

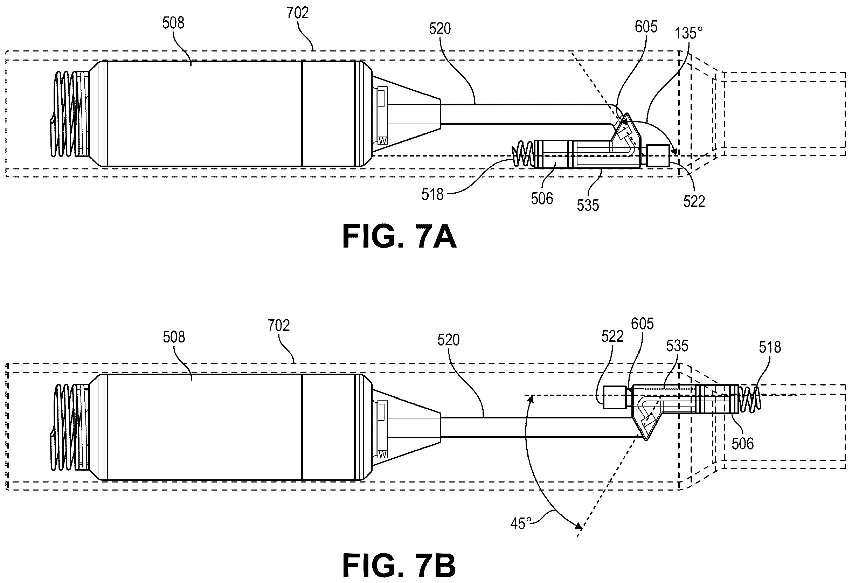

The non-coaxial relationship of the electrode axis and the housing axis, which allows for safe and non-obstructive placement of the pacing electrode 506 and the housing 508, may be provided by an electrical extension 520 of the biostimulator 500. The electrical extension 520 can be located between the pacing extension body 535 and the housing 508. For example, the electrical extension 520 may include an elongated electrical conductor to couple the pacing electrode 506 of the pacing extension body 535 with the electronics of the housing 508. The electrical extension 520 may extend from a proximal extension end at the housing to a distal extension end where the pacing extension body 535 is mounted (e.g., at mounting point 534). More particularly, electrical extension 520 may include a flexible tubing, conduit, casing, molding, etc. to provide electrical isolation or encasing of the elongated electrical conductor. In some examples, as depicted in FIG. 5, the electrical extension 520 may be mounted to the pacing extension body 535 at an angle at approximately 45 degrees. In some examples, the electrical extension 520 may be mounted to the pacing extension body 535 at an angle ranging, for example, from 45 degrees to 135 degrees. However, the angle is not limited to such and may be mounted at any other angle. As described in further detail below with respect to FIGS. 7A and 7B, the angle at which the electrical extension 520 is coupled to the pacing extension body 535 may determine an orientation and method of loading the biostimulator 500 into a delivery or transport system.

Leadless pacemakers or other leadless biostimulators 500 can be delivered to or retrieved from a patient using delivery or retrieval systems. The leadless biostimulator system can include delivery or retrieval systems, which may be catheter-based systems used to carry a leadless biostimulator 500 intravenously to or from a patient anatomy. The delivery or retrieval systems may be referred to collectively as transport systems, or biostimulator transport systems. Examples of transport systems are described below. In some implementations of biostimulator systems, a leadless pacemaker is attached, connected to, or otherwise mounted on a distal end of a catheter of the biostimulator transport system. The leadless pacemaker is thereby advanced intravenously into or out of the heart 102. The transport system can include features to engage the leadless pacemaker to allow fixation of the leadless pacemaker to tissue. For example, in implementations where the leadless pacemaker includes an active engaging mechanism, such as a helical fixation element, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 500 is delivered to and screwed into the septum of the heart 102, the pacing electrode 506 may be positioned for deep septal pacing at a target bundle branch 122 in the septum. For example, an active electrode of the pacing element can be positioned at the left bundle branch 122 in the septum. The biostimulator 500 may deliver pacing impulses through the pacing electrode 506 to the bundle branch(es).

Referring to FIG. 6, a side view of a pacing extension body of a biostimulator having an integrated drive mechanism and a free-rotating fixation helix is shown in accordance with an embodiment. The pacing extension body 535 includes an integrated torque drive 522, a pacing electrode 506, and fixation helix 518. The fixation helix 518 may be freely rotating within the pacing electrode 506. The electrical extension 520 may be coupled to the pacing extension body 535 at mount point 605. For example, the electrical extension 520 may be comprised of a flexible material and may be attached to the pacing extension body 535 at mount point 605, the pacing extension body 535 being comprised of a hard plastic, metal, or other inflexible material. The fixation helix 518 may be mounted at a distal end of the pacing extension body 535, proximate to the pacing electrode 506. The fixation helix 518 may rotate freely upon application of torque to the integrated torque drive 522. For example, the torque drive 522 may also be freely rotatable within the pacing extension body 535 and may be directly coupled with the fixation helix 518.

Referring to FIG. 7A, a side view of a biostimulator having a pacing extension body with an integrated drive mechanism and free-rotating fixation helix in a transport system is shown in accordance with an embodiment. To deploy the biostimulator 500, the biostimulator 500 may be inserted within a transport system 702, such as within a catheter that may be used to deploy the biostimulator intravenously to the heart. To incorporate the biostimulator with the electrical extension 520, housing 508, and the pacing extension body 535 within the transport system 702, the electrical extension 520 may be folded, articulated, or bent within the transport system 702. For example, the electrical extension 520 may be bent at or near the mounting point 605 between the electrical extension 520 and the pacing extension body 535 to be inserted into the transport system 702. The angle between the electrical extension 520 and the pacing extension body 535 at the mounting point 605 may reduce the stress, pinching, or both on the elongated electrical conductor disposed in the electrical extension 520 when folded and inserted within the transport system 702. In particular, for a mounting angle of the pacing extension body 535 with the electrical extension 520 at 135 degrees (or any mounting angle in the approximate range of 90 degrees to 135 degrees), the biostimulator may be loaded into the transport system 702 as depicted in FIG. 7A. In some embodiments, the transport system 702 may be used to first attach the housing 508 (e.g., at a ventricular apex 105) after which the biostimulator 500 may be removed from the transport system 702. The fixation helix 518 and pacing electrode 506 of the pacing extension body 535 may then be aligned and attached (e.g., at a septal wall 104). The torque drive 522 may be used to apply a torque to rotate the fixation helix 518 until an appropriate depth of the pacing electrode 506 is reached within the septal wall.

Referring to FIG. 7B, a side view of a biostimulator having a pacing extension body with an integrated drive mechanism and free-rotating fixation helix in a transport system is shown in accordance with an embodiment. As described above, to deploy the biostimulator 500, the biostimulator 500 may be inserted within a transport system 702, such as within a catheter that may be used to deploy the biostimulator intravenously to the heart. To incorporate the biostimulator with the electrical extension 520, housing 508, and the pacing extension body 535 within the transport system 702, the electrical extension 520 may be folded, articulated, or bent within the transport system 702. For example, the electrical extension 520 may be bent at or near the mounting point 605 between the electrical extension 520 and the pacing extension body 535 to be inserted into the transport system 702. The angle between the electrical extension 520 and the pacing extension body 535 at the mounting point 605 may reduce the stress, pinching, or both on the elongated electrical conductor disposed in the electrical extension 520 when folded and inserted within the transport system 702. In particular, for a mounting angle of the pacing extension body 535 with the electrical extension 520 at 45 degrees (or any mounting angle in the approximate range of 45 degrees to 90 degrees), the biostimulator may be loaded into the transport system 702 as depicted in FIG. 7B.

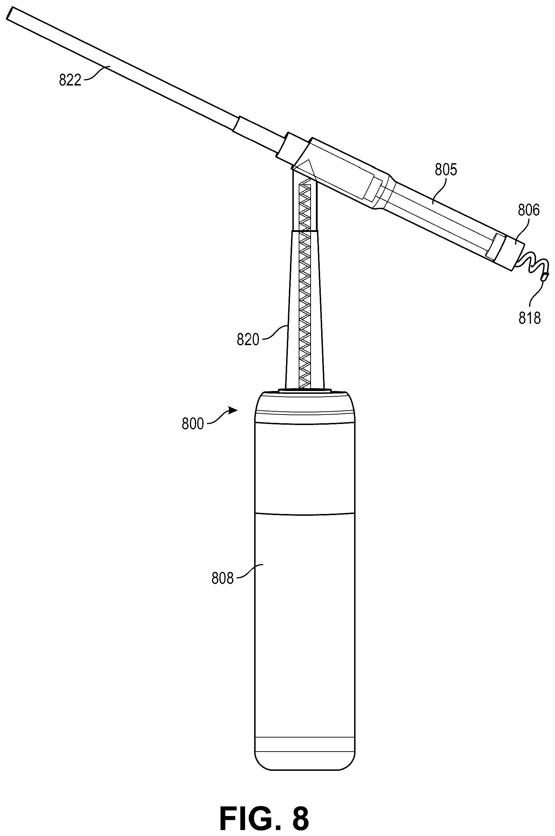

Referring to FIG. 8, a side view of a biostimulator including an angled electrode extension with a free-rotating fixation helix is shown in accordance with an embodiment. The biostimulator 800 includes a housing 808 including pacing circuitry. An electrical extension 820 may extend from the housing and may include an elongated electrical conductor to connect the pacing circuity to the pacing electrode. A pacing extension body 805 may include a pacing electrode 806 and a free-rotating fixation helix 818. The pacing extension body 805 may further include an integrated torque drive 822 for applying torque to the fixation helix 818. In some examples, the torque drive may be removable or separable from the pacing extension body 805.

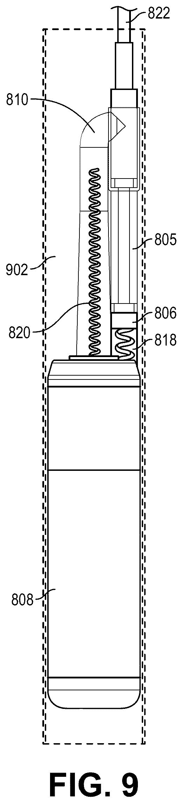

The electrical extension 820 may extend straight (e.g., on the same axis) as the housing 808. The pacing extension body 805 may be coupled with the electrical extension 820 at an obtuse angle with respect to the axis of the electrical extension 820 (e.g., at around 135 degrees). In some examples, a semi-flexible material may be molded over the electrical extension 820 and the pacing extension body 805 to provide structural support to the device and maintain the predetermined angle to best achieve LBB pacing while keeping the device flexible enough to be bent to fit inside a deliver catheter, as depicted in FIG. 9.

Referring to FIG. 9, a side view of a biostimulator including an angled electrode extension with a free-rotating fixation helix in a transport system is shown in accordance with an embodiment. The transport system 702 can include a delivery catheter 902. The delivery catheter 902 may, for example, include a protective sleeve having an inner lumen to receive the biostimulator 800, including the housing 808 and the bent pacing extension body 805. The protective sleeve may be tubular, and may extend from a distal portion having a cavity to receive the biostimulator 800 to a proximal end that may be manipulated by a user. For example, the user may push or pull the proximal end to cause the protective sleeve to cover or release the biostimulator 800. The biostimulator 800 may therefore move between the constrained state shown in FIG. 9 to the unconstrained state shown in FIG. 8.

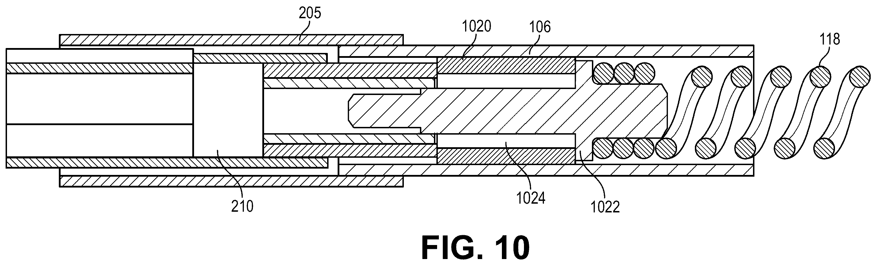

Referring to FIG. 10, a cross-sectional view of a pacing extension body with a free-rotating fixation helix is shown in accordance with an embodiment. The pacing extension body 205 may include a helix mount 1022 to which a fixation helix 118 is attached or mounted. A collar 1020 may be included within a pacing electrode 106. The collar 1020 may couple the helix mount 1022 of the fixation helix 118 to the pacing extension body 205 and allow the helix mount 1022 and the fixation helix to freely rotation within the pacing extension body 205. Additionally, a torque drive coupler 1024 may be attached to the helix mount 1022 and may engage with a torque drive 210. For example, the torque drive 210, which may be a wrench, may be inserted into a drive port 112 inside the pacing extension body 205. The torque drive 210 can engage a socket of the torque drive coupler 1024, and apply a torque to the fixation helix 118 through the coupler 1024 and the helix mount 1022.

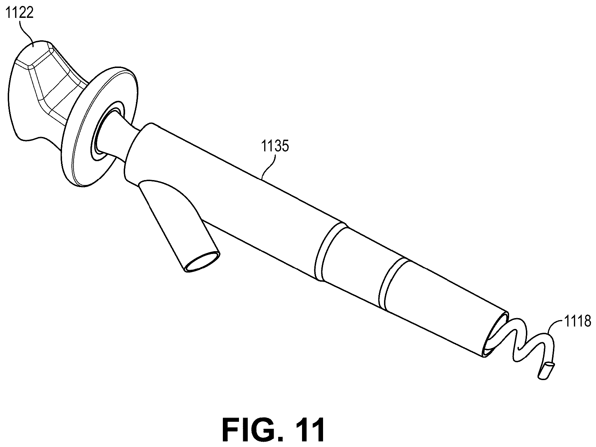

Referring to FIG. 11, a perspective view of a pacing extension body with a free-rotating fixation helix with attachment and detachment button is shown in accordance with an embodiment. In some embodiments, the pacing extension body 1135 including a free-rotating fixation helix 1118 may include a leadless pacemaker (LP) button 1122 to apply torque to spin the free-rotating fixation helix 1118, rather than a torque driver. The LP button 1122 may replace the torque drive socket (e.g., as described with respect to FIGS. 1-9) and adds a button feature that may correspond to other commercially available torque features. Accordingly, the LP button 1122 may provide compatibility of the free-rotating fixation helix 1118 with standard retrieval style catheters for retrieval of the pacing extension body 1135 and the entire leadless biostimulator.

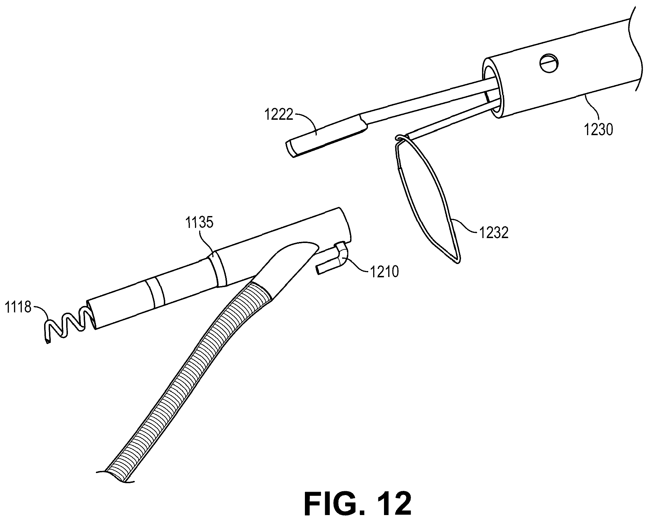

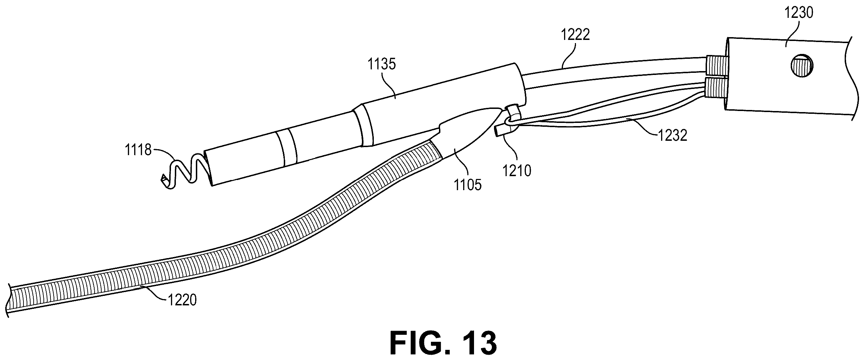

Referring to FIG. 12, a side view of a pacing extension body including a retrieval component and a retrieval system is shown in accordance with an embodiment. In some embodiments, a hook 1210 is located on the pacing extension body 1135. As depicted in FIG. 13, during retrieval of the pacing extension body 1135, a snare 1232 loops around the hook 1210 and captures the pacing extension body 1135. The snare 1232 may provide a constant tension on the system to keep it secured in a protective sleeve 1230 and prevent any spontaneous releases. Additionally, the torque drive 1222 may be inserted into the pacing extension body 1135 to apply a torque to remove the fixation helix 1118 from the attachment point. The figure further shows the electrical extension 1220 and a mounting point 1105 as previously described herein.

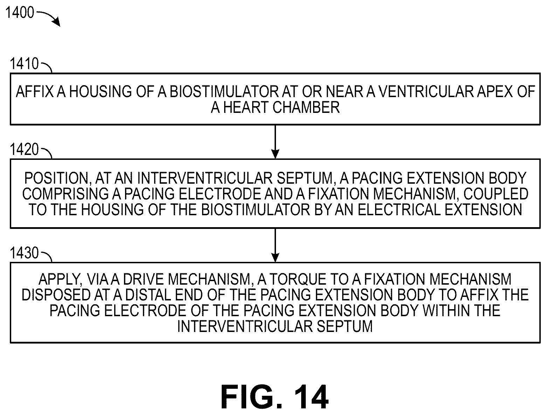

Referring to FIG. 14, a flowchart of a method of implanting a biostimulator for septal pacing is shown. During the implantation procedure, a biostimulator transport system can carry the biostimulator into the target heart chamber. When implantation is to be within the right ventricle, the biostimulator transport system can be tracked through the inferior vena cava into the right atrium and across the tricuspid valve into the right ventricle. The distal end of the transport system can be steered toward a desired location of the septal wall. For example, the target area may be in an upper region of an interventricular septal wall.

At operation 1410, a housing of the biostimulator is affixed at or near a ventricular apex of the target heart chamber. At operation 1420, a pacing extension body is positioned at an interventricular septum, wherein the pacing extension body includes a pacing electrode and a fixation mechanism and is coupled to the housing of the biostimulator by an electrical extension. The housing may include an attachment feature disposed on an end of the housing opposite an electrical extension.

At operation 1430, a torque is applied to the fixation mechanism disposed at a distal end of the pacing extension body to affix the pacing electrode of the pacing extension body within the interventricular septum. wherein the housing and the pacing extension body are affixed using a single deployment tool. In some examples, the fixation mechanism is a fixation helix. In some embodiments, the pacing electrode includes an electrode ring disposed on the pacing extension body proximal to the fixation helix. The fixation helix may be freely rotatable within the pacing extension body. In some embodiments, pacing electrode, the fixation helix, and the drive mechanism may be integrated together in the pacing extension body. In some embodiments, the electrical extension includes a first semi-flexible portion extending from the housing and a second portion coupled to the first semi-flexible portion at an obtuse angle with respect to the first semi-flexible portion at an attachment point between the first semi-flexible portion and second portion. Once the pacing extension body is affixed within the interventricular septum, the pacing circuitry within the housing may deliver a pacing impulse at various intervals to the target tissue (e.g., the left bundle branch) through the pacing electrode of the pacing extension body.

An aspect of the disclosure relates to a biostimulator 100, 500, 800 comprising a housing 108, 508, 808 having an electronics compartment containing pacing circuitry. The biostimulator 100, 500, 800 also comprises an electrical extension 120, 520, 820 having an elongated electrical conductor 215. The electrical extension 120, 520, 820 extends from a proximal extension end at the housing 108, 508, 808 to a distal extension end. The biostimulator 100, 500, 800 further comprises a pacing extension body 205, 535, 805, 1135 mounted on the distal extension end of the electrical extension 120, 520, 820. The pacing extension body 205, 535, 805, 1135 includes a pacing electrode 106, 506, 806 electrically coupled to the pacing circuitry through the elongated electrical conductor 215. The pacing extension body 205, 535, 805, 1135 also comprises a fixation mechanism 118, 518, 818, 1118, and a drive mechanism to transmit torque to the fixation mechanism 118, 518, 818, 1118.

In an embodiment, the fixation mechanism 118, 518, 818, 1118 comprises a fixation helix 118, 518, 818, 1118.

In an embodiment, the fixation helix 118, 518, 818, 1118 is free turning within the pacing extension body 205, 535, 805, 1135.

In an embodiment, the pacing extension body 205, 535, 805, 1135 comprises a port 112 providing access for insertion of a drive tool 210, 522, 822, 1222 to apply a torque to the drive mechanism of the pacing extension body 205, 535, 805, 1135.

In an embodiment, the port 112 includes a torque drive socket.

In an embodiment, the pacing electrode 106, 506, 806 comprises an electrode ring disposed on the pacing extension body 205, 535, 805, 1135 proximal to the fixation helix 118, 518, 818, 1118.

In an embodiment, the drive tool 822 is integrated in the pacing extension body 805.

In an embodiment, the housing 108, 508, 808 comprises an attachment feature 110, 510 disposed on an end of the housing 108, 508, 808 opposite the electrical extension 120.

In an embodiment, the electrical extension 120, 520, 820 comprises a first semi-flexible portion extending from the housing 108, 508, 808.

In an embodiment, the electrical extension 120, 520, 820 comprises a second portion coupled to the first semi-flexible portion at an obtuse angle with respect to the first semi-flexible portion at an attachment point between the first semi-flexible portion and the second portion.

In an embodiment, the pacing extension body 205, 535, 805, 1135 further comprises a release and retrieval mechanism 1210.

In an embodiment, the release and retrieval mechanism 1210 includes a hook 1210.

Another aspect of the disclosure relates to a biostimulator system comprising a biostimulator transport system 402, 702, 902, and a biostimulator 100, 500, 800 according to the disclosure mounted on the biostimulator transport system 402, 702, 902.

In an embodiment, the biostimulator transport system 402, 702, 902 comprises a catheter 402, 702, 902. The electrical extension 120, 520, 820 is configured to transition from a delivery state when the elongated extension 120, 520, 820 is stored in the catheter 402, 702, 902 to a deployed state when the elongated extension 120, 520, 820 is exposed from the catheter 402, 702, 902.

In an embodiment, an angle between the electrical extension 120, 520, 720 and the pacing extension body 205, 525, 805, 1135 is greater in the deployed state than in the delivery state.

## Claims

1. A biostimulator (100, 500, 800), comprising:
a housing (108, 508, 808) having an electronics compartment containing pacing circuitry;
an electrical extension (120, 520, 820) having an elongated electrical conductor (215), wherein the electrical extension (120, 520, 820) extends from a proximal extension end at the housing (108, 508, 808) to a distal extension end and wherein the electrical extension (120, 520, 820) is flexible to bend and move the distal extension end relative to the proximal extension end; and
a pacing extension body (205, 535, 805, 1135) mounted on the distal extension end of the electrical extension (120, 520, 820), wherein the pacing extension body (205, 535, 805, 1135) includes:
a pacing electrode (106, 506, 806) electrically coupled to the pacing circuitry through the elongated electrical conductor (215), and
a free-rotating fixation helix (118, 518, 818, 1118), **characterized in that** the pacing extension body (205, 535, 805, 1135) further includes:
a drive mechanism to transmit torque to rotate the free-rotating fixation helix (118, 518, 818, 1118) relative to the electrical extension (120, 520, 820) and the housing (108, 508, 808).

2. The biostimulator of claim 1, wherein the pacing extension body (205, 535, 805, 1135) comprises a port (112) providing access for insertion of a drive tool (210, 522, 822, 1222) to apply a torque to the drive mechanism of the pacing extension body (205, 535, 805, 1135).

3. The biostimulator of claim 2, wherein the port (112) includes a torque drive socket.

4. The biostimulator of claim 2 or 3, wherein the pacing electrode (106, 506, 806) comprises an electrode ring disposed on the pacing extension body (205, 535, 805, 1135) proximal to the free-rotating fixation helix (118, 518, 818, 1118).

5. The biostimulator of any one of claims 2 to 4, wherein the drive tool (822) is integrated in the pacing extension body (805).

6. The biostimulator of any one of claims 1 to 5, wherein the housing (108, 508, 808) comprises an attachment feature (110, 510) disposed on an end of the housing (108, 508, 808) opposite the electrical extension (120).

7. The biostimulator of any one of claims 1 to 6, wherein the electrical extension (120, 520, 820) comprises a first semi-flexible portion extending from the housing (108, 508, 808).

8. The biostimulator of claim 7, wherein the electrical extension (120, 520, 820) comprises a second portion coupled to the first semi-flexible portion at an obtuse angle with respect to the first semi-flexible portion at an attachment point between the first semi-flexible portion and the second portion.

9. The biostimulator of any one of claims 1 to 8, wherein the pacing extension body (205, 535, 805, 1135) further comprises a release and retrieval mechanism (1210).

10. The biostimulator of claim 9, wherein the release and retrieval mechanism (1210) includes a hook (1210).

11. A biostimulator system comprising:
a biostimulator transport system (402, 702, 902); and
a biostimulator (100, 500, 800) of any one of claims 1 to 10 mounted on the biostimulator transport system (402, 702, 902).

12. The biostimulator system of claim 11, wherein the biostimulator transport system (402, 702, 902) comprises a catheter (402, 702, 902), wherein the electrical extension (120, 520, 820) is configured to transition from a delivery state when the elongated extension (120, 520, 820) is stored in the catheter (402, 702, 902) to a deployed state when the elongated extension (120, 520, 820) is exposed from the catheter (402, 702, 902).

13. The biostimulator system of claim 12, wherein an angle between the electrical extension (120, 520, 720) and the pacing extension body (205, 525, 805, 1135) is greater in the deployed state than in the delivery state.

## Patentansprüche

1. Biostimulator (100, 500, 800), umfassend:
ein Gehäuse (108, 508, 808), das ein Elektronikfach aufweist, das eine Stimulationsschaltung enthält;
eine elektrische Verlängerung (120, 520, 820), die einen länglichen elektrischen Leiter (215) aufweist, wobei sich die elektrische Verlängerung (120, 520, 820) von einem proximalen Verlängerungsende an dem Gehäuse (108, 508, 808) zu einem distalen Verlängerungsende erstreckt und wobei die elektrische Verlängerung (120, 520, 820) flexibel ist, um das distale Verlängerungsende relativ zu dem proximalen Verlängerungsende zu biegen und zu bewegen; und
einen Stimulationsverlängerungskörper (205, 535, 805, 1135), der an dem distalen Verlängerungsende der elektrischen Verlängerung (120, 520, 820) montiert ist, wobei der Stimulationsverlängerungskörper (205, 535, 805, 1135) Folgendes beinhaltet:
eine Stimulationselektrode (106, 506, 806), die mit der Stimulationsschaltung durch den länglichen elektrischen Leiter (215) elektrisch gekoppelt ist, und
eine frei drehende Fixierungshelix (118, 518, 818, 1118), **dadurch gekennzeichnet, dass** der Stimulationsverlängerungskörper (205, 535, 805, 1135) ferner Folgendes beinhaltet:
einen Antriebsmechanismus, um Drehmoment zu übertragen, um die frei drehende Fixierungshelix (118, 518, 818, 1118) relativ zu der elektrischen Verlängerung (120, 520, 820) und dem Gehäuse (108, 508, 808) zu drehen.

2. Biostimulator nach Anspruch 1, wobei der Stimulationsverlängerungskörper (205, 535, 805, 1135) einen Anschluss (112) umfasst, der Zugang zum Einsetzen eines Antriebswerkzeugs (210, 522, 822, 1222) bereitstellt, um ein Drehmoment auf den Antriebsmechanismus des Stimulationsverlängerungskörpers (205, 535, 805, 1135) anzuwenden.

3. Biostimulator nach Anspruch 2, wobei der Anschluss (112) eine Drehmomentantriebsaufnahme beinhaltet.

4. Biostimulator nach Anspruch 2 oder 3, wobei die Stimulationselektrode (106, 506, 806) einen Elektrodenring umfasst, der an dem Stimulationsverlängerungskörper (205, 535, 805, 1135) proximal zu der frei drehenden Fixierungshelix (118, 518, 818, 1118) angeordnet ist.

5. Biostimulator nach einem der Ansprüche 2 bis 4, wobei das Antriebswerkzeug (822) in den Stimulationsverlängerungskörper (805) integriert ist.

6. Biostimulator nach einem der Ansprüche 1 bis 5, wobei das Gehäuse (108, 508, 808) ein Anbringungsmerkmal (110, 510) umfasst, das an einem Ende des Gehäuses (108, 508, 808) gegenüber der elektrischen Verlängerung (120) angeordnet ist.

7. Biostimulator nach einem der Ansprüche 1 bis 6, wobei die elektrische Verlängerung (120, 520, 820) einen ersten halbflexiblen Abschnitt umfasst, der sich von dem Gehäuse (108, 508, 808) erstreckt.

8. Biostimulator nach Anspruch 7, wobei die elektrische Verlängerung (120, 520, 820) einen zweiten Abschnitt umfasst, der mit dem ersten halbflexiblen Abschnitt in einem stumpfen Winkel in Bezug auf den ersten halbflexiblen Abschnitt an einem Anbringungspunkt zwischen dem ersten halbflexiblen Abschnitt und dem zweiten Abschnitt gekoppelt ist.

9. Biostimulator nach einem der Ansprüche 1 bis 8, wobei der Stimulationsverlängerungskörper (205, 535, 805, 1135) ferner einen Freigabe- und Rückholmechanismus (1210) umfasst.

10. Biostimulator nach Anspruch 9, wobei der Freigabe- und Rückholmechanismus (1210) einen Haken (1210) beinhaltet.

11. Biostimulatorsystem, umfassend:
ein Biostimulatortransportsystem (402, 702, 902); und
einen Biostimulator (100, 500, 800) nach einem der Ansprüche 1 bis 10, der an dem Biostimulatortransportsystem (402, 702, 902) montiert ist.

12. Biostimulatorsystem nach Anspruch 11, wobei das Biostimulatortransportsystem (402, 702, 902) einen Katheter (402, 702, 902) umfasst, wobei die elektrische Verlängerung (120, 520, 820) dazu konfiguriert ist, von einem Lieferzustand, wenn die längliche Verlängerung (120, 520, 820) in dem Katheter (402, 702, 902) untergebracht ist, in einen ausgefahrenen Zustand, wenn die längliche Verlängerung (120, 520, 820) aus dem Katheter (402, 702, 902) freigelegt ist, überzugehen.

13. Biostimulatorsystem nach Anspruch 12, wobei ein Winkel zwischen der elektrischen Verlängerung (120, 520, 720) und dem Stimulationsverlängerungskörper (205, 525, 805, 1135) in dem ausgefahrenen Zustand größer ist als in dem Lieferzustand.

## Revendications

1. Biostimulateur (100, 500, 800), comprenant :
un boîtier (108, 508, 808) ayant un compartiment électronique contenant une circuiterie de stimulation ;
un prolongement électrique (120, 520, 820) ayant un conducteur électrique allongé (215), dans lequel le prolongement électrique (120, 520, 820) s'étend à partir d'une extrémité de prolongement proximale sur le boîtier (108, 508, 808) jusqu'à une extrémité de prolongement distale et dans lequel le prolongement électrique (120, 520, 820) est flexible pour fléchir et déplacer l'extrémité de prolongement distale par rapport à l'extrémité de prolongement proximale ; et
un corps de prolongement de stimulation (205, 535, 805, 1135) monté sur l'extrémité de prolongement distale du prolongement électrique (120, 520, 820), dans lequel le corps de prolongement de stimulation (205, 535, 805, 1135) inclut :
une électrode de stimulation (106, 506, 806) couplée électriquement à la circuiterie de stimulation par l'intermédiaire du conducteur électrique allongé (215), et
une hélice de fixation à rotation libre (118, 518, 818, 1118), **caractérisé en ce que** le corps de prolongement de stimulation (205, 535, 805, 1135) inclut en outre :
un mécanisme d'entraînement pour transmettre un couple afin de faire tourner l'hélice de fixation à rotation libre (118, 518, 818, 1118) par rapport au prolongement électrique (120, 520, 820) et au boîtier (108, 508, 808).

2. Biostimulateur selon la revendication 1, dans lequel le corps de prolongement de stimulation (205, 535, 805, 1135) comprend un orifice (112) fournissant un accès pour l'insertion d'un outil d'entraînement (210, 522, 822, 1222) pour appliquer un couple au mécanisme d'entraînement du corps de prolongement de stimulation (205, 535, 805, 1135).

3. Biostimulateur selon la revendication 2, dans lequel l'orifice (112) inclut une douille d'entraînement de couple.

4. Biostimulateur selon la revendication 2 ou 3, dans lequel l'électrode de stimulation (106, 506, 806) comprend une bague d'électrode disposée sur le corps de prolongement de stimulation (205, 535, 805, 1135) de manière proximale par rapport à l'hélice de fixation à rotation libre (118, 518, 818, 1118).

5. Biostimulateur selon l'une quelconque des revendications 2 à 4, dans lequel l'outil d'entraînement (822) est intégré dans le corps de prolongement de stimulation (805).

6. Biostimulateur selon l'une quelconque des revendications 1 à 5, dans lequel le boîtier (108, 508, 808) comprend un élément de fixation (110, 510) disposé sur une extrémité du boîtier (108, 508, 808) opposée au prolongement électrique (120).

7. Biostimulateur selon l'une quelconque des revendications 1 à 6, dans lequel le prolongement électrique (120, 520, 820) comprend une première partie semi-flexible s'étendant à partir du boîtier (108, 508, 808).

8. Biostimulateur selon la revendication 7, dans lequel le prolongement électrique (120, 520, 820) comprend une seconde partie couplée à la première partie semi-flexible en formant un angle obtus par rapport à la première partie semi-flexible au niveau d'un point de fixation entre la première partie semi-flexible et la seconde partie.

9. Biostimulateur selon l'une quelconque des revendications 1 à 8, dans lequel le corps de prolongement de stimulation (205, 535, 805, 1135) comprend en outre un mécanisme de libération et de récupération (1210).

10. Biostimulateur selon la revendication 9, dans lequel le mécanisme de libération et de récupération (1210) inclut un crochet (1210).

11. Système de biostimulateur comprenant :
un système de transport de biostimulateur (402, 702, 902) ; et
un biostimulateur (100, 500, 800) selon l'une quelconque des revendications 1 à 10 monté sur le système de transport de biostimulateur (402, 702, 902).

12. Système de biostimulateur selon la revendication 11, dans lequel le système de transport de biostimulateur (402, 702, 902) comprend un cathéter (402, 702, 902), dans lequel le prolongement électrique (120, 520, 820) est configuré pour passer d'un état de mise en place lorsque le prolongement électrique (120, 520, 820) est stocké dans le cathéter (402, 702, 902) à un état déployé lorsque le prolongement électrique (120, 520, 820) est exposé à partir du cathéter (402, 702, 902).

13. Système de biostimulateur selon la revendication 12, dans lequel un angle entre le prolongement électrique (120, 520, 720) et le corps de prolongement de stimulation (205, 525, 805, 1135) est plus grand dans l'état déployé que dans l'état de mise en place.
